(19) Europäisches Patentamt

European Patent Office

Office européen des brevets

(11) **EP 4 721 686 A1**

(12) **EUROPEAN PATENT APPLICATION**

(43) Date of publication:
08.04.2026 Bulletin 2026/15

(21) Application number: 25206586.7

(22) Date of filing: 03.10.2025

(51) International Patent Classification (IPC):
*A61B 18/14* (2006.01)　　*A61B 5/00* (2006.01)
*A61B 17/00* (2006.01)

(52) Cooperative Patent Classification (CPC):
**A61B 18/1492; A61B 5/6885; A61B 17/00;**
A61B 2018/00267; A61B 2090/064; A61B 2090/065

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 04.10.2024 US 202418906402

(71) Applicant: **Biosense Webster (Israel) Ltd.**
**2066717 Yokneam (IL)**

(72) Inventors:
• **BAR-TAL, Meir**
**2066717 Yokneam (IL)**
• **OSADCHY, Daniel**
**2066717 Yokneam (IL)**
• **BOTZER, Lior**
**2066717 Yokneam (IL)**

(74) Representative: **Carpmaels & Ransford LLP**
**One Southampton Row**
**London WC1B 5HA (GB)**

(54) **DEFORMATION BASED PRESSURE SENSING**

(57) A method for performing a medical procedure, consisting of providing a probe, having a known spring constant, for insertion into a patient, the probe having electrodes and a position sensor at respective locations along the probe, and recording relative location coordinates of the electrodes and the position sensor when the probe is in an unrestrained state. While the probe is in the patient and deformed by contact with patient tissue, recording changes in the relative location coordinates of the electrodes in contact with the tissue and the position sensor, and computing a probe shape in a deformed state, in response to the recorded changes. In response to the known spring constant and the computed probe shape, computing a respective force exerted by the tissue on each of the electrodes, and controlling electrical signals through the electrodes from the tissue responsively to the computed respective force on the electrodes.

EP 4 721 686 A1

**Description**

**FIELD OF THE DISCLOSURE**

This disclosure relates generally to catheterization, and specifically to estimating the force exerted by a catheter when it is inserted into tissue.

**BACKGROUND**

A cardiac catheter procedure typically involves initial mapping of the heart of the patient, prior to a operation on the heart such as ablation of cardiac tissue. Both the mapping and the ablation may be performed by electrodes attached to a catheter that is inserted into the heart.

**BRIEF DESCRIPTION OF THE DRAWINGS**

The present disclosure will be understood from the following detailed description, taken in conjunction with the drawings in which:

Fig. 1 shows a catheter-based electrophysiology mapping and ablation system, according to an example of the present disclosure;

Fig. 2 is a flowchart of steps taken by a processor of the system of Fig. 1 in implementing an algorithm to determine a force exerted by a probe, according to an example of the present disclosure; and

Fig. 3 is a schematic illustration of a part of the probe, according to an example of the present disclosure.

**DESCRIPTION OF EXAMPLES**

OVERVIEW

A cardiac catheterization procedure may typically involve both mapping of a region of cardiac tissue, and ablation of a selected section of the tissue, using a multi-electrode assembly attached to a distal end of the catheter. Each electrode of the assembly may be used for mapping and/or ablation of the tissue contacted by the electrode. For any given electrode, recording the position of the electrode when performing the mapping depends on a processor identifying that there is contact with the tissue. In the case of ablation, the dimensions of the ablated tissue depend on the force applied by the electrode to the tissue being ablated (this force is equal and opposite to the force applied by the tissue to the given electrode). While there may be one or more force sensors attached to the assembly, these sensors are insufficient to provide values of the force for individual electrodes of the probe.

The assembly may have one or more splines, also herein termed probes, each of which typically has multiple electrodes. As is described below, examples of the present disclosure provide an estimate of the force from each electrode, as well as an estimate of the pressure (as a force per linear length) on a given probe.

Examples of the present disclosure record the change of shape of a given probe of the assembly, when the probe is deformed by one or more electrodes contacting tissue. The change in shape may be recorded, for example, from signals provided by location sensors incorporated in the probe, and/or from signals generated in a conductive loop carried on or embedded in the probe. Examples of the present disclosure also ascertain which electrodes are touching tissue, and which are not touching, for example using an impedance-based system.

In a disclosed example a processor models a probe as a number of sections connected at joints, and the electrodes of the probe are assumed to be at known locations on the sections. The joints act as torsion springs having a known torsional spring constant, and the sections exert torsional and compression forces on each joint. The magnitudes and directions of the torsional forces and the directions of the compression forces are known from the change of shape of the probe, and from the joint spring constant.

The model divides the joints into two types: a first type that touches the tissue, and a second type that does not touch the tissue. Both types may have external forces on the joints, apart from the torsional and compression forces from the sections that are described above. Each external force is resolved into two orthogonal components: a component parallel to the probe at the joint, and a component at right angles to the probe.

The model assumes that the first type of joints (those contacting the tissue) are "slippery," so that the parallel components of the external forces, from the tissue contacting the joints, are minimal. In addition, since the second type of joints do not contact the tissue, the right angle components of the external forces of the second type are also minimal.

The processor uses a minimization function to calculate values of all components of all the joint external forces. In evaluating the minimization function, the processor uses the known joint torsional values and also applies a constraint that, since the probe is in equilibrium, the total of all the forces at each joint is zero.

From the calculated component values, the processor computes the external force on each of the joints, and may interpolate the forces to calculate the pressure on each section of the probe. The processor may use the calculated pressure to find the force on each electrode of the probe.

As explained above, any given electrode of the probe may be used for mapping contacted tissue, the mapping being implemented by the processor measuring signals received from the electrode and evaluating a location for the electrode in response to the signals. The processor may be configured to accept the evaluated location when the evaluated force on the electrode from the tissue (which is the same as the force on the tissue from the electrode) is greater than a preset minimum force.

Alternatively or additionally, any given electrode may be used for ablating tissue. The processor may be configured to adjust the duration and/or value of the ablation current through the electrode in response to the evaluated force on the tissue from the electrode.

SYSTEM DESCRIPTION

In the following description, like elements are identified by the same numeral, and are differentiated, where required, by having a letter attached as a suffix to the numeral.

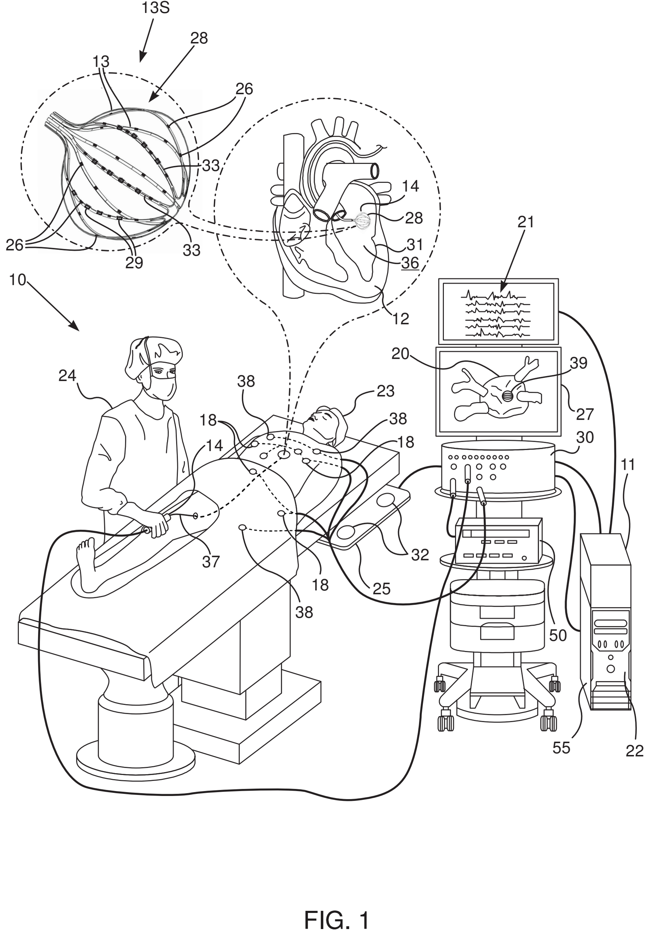

Reference is now made to Fig. 1, which shows a catheter-based electrophysiology mapping and ablation system 10 being used for a medical procedure, according to an example of the present disclosure. System 10 includes multiple catheters, which are percutaneously inserted by a physician 24 through the patient's vascular system into a chamber or vascular structure of a heart 12 of a patient 23. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating, and/or catheters dedicated for both sensing and ablating.

An example catheter 14 that is configured for a sensing and/or an ablation procedure is illustrated herein, the catheter comprising an insertion shaft 37 and a distal end assembly 28 fixed to a distal end of the shaft. During the procedure, once distal end assembly 28 exits the delivery sheath, physician 24 manipulates a proximal end of shaft 37, so as to bring the assembly into contact with a heart wall 31 of a chamber 36 of heart 12, for the purpose of sensing signals from, or ablating, a target site in the wall.

In the description herein, except where otherwise stated, distal end assembly 28 is a basket that is constructed as a plurality of similar resilient splines or probes 13. Probes 13 form assembly 28 to have a generally spheroidal shape and act as a supporting structure 13S for attached electrodes 26.

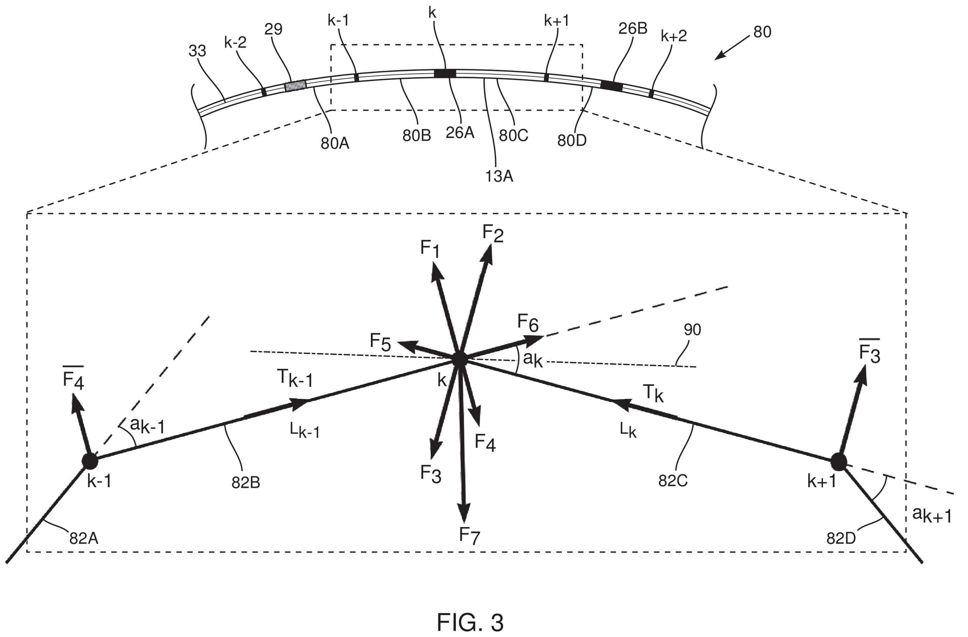

Each of the probes of structure 13S has at least one attached electrode 26 which may be used for signal sensing and/or ablation. Each probe has a known length, and each attached electrode 26 is in a known location on its respective probe. At least one position sensor 29 is also attached to each probe 13, the position sensors being substantially similar to each other. Position sensors 29 are typically magnetic based position sensors, having at least one coil, and are typically multiple coils having orthogonal axes. A given sensor generates signals indicative of the location and orientation of the sensor, in response to magnetic fields traversing the sensor. The magnetic fields are generated by a plurality of magnetic coils 32 positioned in a location pad 25 in proximity to assembly 28. Fig. 3, referred to in more detail below, includes an enlarged schematic illustration of a probe 13A, together with its electrodes 26, position sensor 29, and an attached linear conductor 33, described below.

Details of magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

In a disclosed example at least one probe 13 has a respective linear conductor 33 attached to the length of the probe, and the conductor acts as an open loop; in some configurations two conductors 33 on separate probes may be connected together, typically at a distal end of the conductors, so that the open loop is substantially closed. The magnetic fields from coils 32 traversing conductors 33 generate signals between the terminations of the open loop formed by the conductors, and these signals are indicative of the shape of the probes to which the conductors are attached.

System 10 includes one or more electrode patches 38 positioned for skin contact on patient 23. Measurements of the impedance between patches 38 and a given electrode 26 may be used determine a location of the electrode as well as to identify if the electrode is contacting tissue of wall 31. U.S. Patent 11,596,324 describes how the impedance between an electrode on a basket catheter and patches on the skin of a patient may be used to identify if the electrode is contacting tissue in a chamber of an organ of the patient. Optionally, impedance between intracardiac electrodes may be sensed to assess contact with the cavity wall.

A recorder 11 displays electrograms 21 captured with body surface ECG electrodes 18 and intracardiac electrograms (IEGM) that may be captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE).

A patient interface unit (PIU) 30 is an interface configured to establish electrical communication between catheters, electrophysiological equipment, a power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally, PIU 30 additionally includes processing capability for implementing real-time computations of location of the catheters and for performing ECG calculations.

Workstation 55 includes memory, a processor 22 with memory or storage with appropriate operating software loaded therein, and user interface capability. Processor 22 operates system 10. Workstation 55 may provide multiple functions, optionally including (1) modeling the endocardial anatomy in three-dimensions (3D) and rendering a model or anatomical map 20 of heart 12 or a portion thereof for display on a display device 27, (2) displaying on display device 27 activation sequences (or other data) compiled from recorded electrograms 21 in representative visual indicia or imagery superimposed on the rendered anatomical map 20, (3) displaying a representation 39, incorporating real-time location and orientation values, of distal end assembly 28 within heart chamber 36, and (4) displaying on display device 27 sites of interest such as places where ablation energy has been applied. One commercial product embodying elements of the system 10 is available as the CARTO™ 3 System, available from Biosense Webster, Inc., 31 Technology Drive, Suite 200, Irvine, CA 92618.

As stated above, in the procedure illustrated in Fig. 1, at least some electrodes 26 on a probe 13 may contact tissue of heart 12. Following is a description of an algorithm of how processor 22 determines the force exerted by a selected electrode on the tissue, and the corresponding opposing force exerted by the tissue.

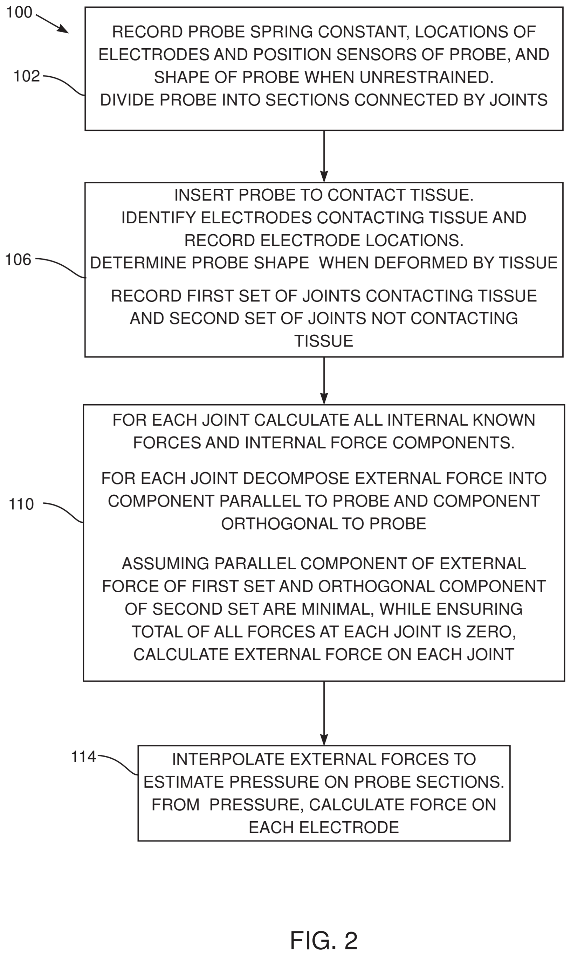

Fig. 2 is a flowchart 100 of steps taken by processor 22 in implementing the algorithm to determine a force exerted by a probe 13A, and Fig. 3 is a schematic illustration of a part of the probe, according to an example of the present disclosure. For simplicity and clarity, in the following description probe 13A is assumed to lie in a two-dimensional (2D) plane, corresponding to the plane of the paper of Fig. 3. Those having ordinary skill in the art will be able to adapt the description, *mutatis mutandis,* for a probe lying in three dimensions.

The algorithm models the probe as being divided into sections having respective terminations, the sections being connected together at their terminations. Each termination is assumed to act as a spring-like joint having a torsional spring constant j, and the terminations are also referred to herein as joints. In the following description the probe is assumed to have N joints, connected by N-1 sections, where N is a positive integer, and the joints are identified by an index k, where k = 1, 2, ... N. In a disclosed example N = 10, but it will be understood that in other examples N may be larger or smaller than 10.

Thus, Fig. 3 illustrates a part of probe 13A having joints k-2, k-1, k, k+1, k+2, and adjacent joints are connected by generally similar sections 80A, 80B, 80C, and 80D, generically termed sections 80. The probe is assumed to have at least one position sensor 29, and at least one electrode 26. By way of example the figure illustrates one position sensor 29 and two electrodes: electrode 26A and electrode 26B. Electrode 26A is shown as coincident with joint k, but in general electrodes and joints of the probe may or may not be coincident.

An enlarged portion of probe 13A, including joints k- 1, k, and k+1, connected by sections 80B and 80C is illustrated in the lower part of the figure. In the lower part sections 80B and 80C have been drawn as straight line connections 82B and 82C, so as to more clearly illustrate the forces acting on the joints.

In an initial step 102 of the flowchart, which may be implemented before the procedure illustrated in Fig. 1 is performed, processor 22 records known parameters of probe 13A. As stated above, the algorithm assumes that the probe behaves as a spring, and the known parameters include the torsional spring constant j of the probe. The parameters also include the locations of electrodes 26, position sensors 29. and conductor 33 on probe 13A.

Processor 22 also records the relative location coordinates of electrodes 26, position sensors 29 and conductor 33, and the processor uses these coordinates to formulate data describing the shape of probe 13A when it is in an unrestrained form, i.e., when there is no external force acting on the probe other than the force due to gravity. The data for the unrestrained shape may be in any convenient form, such as a set of ordered triples and/or an analytic equation.

Processor 22 also divides probe 13A into sections 80 connecting adjacent joints, as described above with reference to Fig. 3, and records the locations of the joints.

In a procedural step 106 physician 24 inserts probe 13A into heart 12 of patient 23 until the probe is located at a desired target location wherein it contacts tissue of the heart, and to assist the physician processor 22 may display representation 39 of the probe on display device 27.

Processor 22 may use the signals from probe sensors 29 and conductor 33 to ascertain that the probe is at the desired location. Processor 22 also measures the impedances between electrodes 26 and patches 38, and uses the impedance measurement signals to calculate the locations of electrodes 26, and to identify electrodes 26 contacting the tissue.

Contact with tissue of heart 12 deforms the shape of probe 13A from its unrestrained shape recorded in initial step 102. In step 106 processor 22 uses the signals from probe sensors 29, conductor 33, and the impedance signals to find positions of portions of probe 13A so as to formulate a deformed shape for the probe.

In step 106 processor 22 identifies a first set $G_1$ of joints that are contacting tissue of heart 12, and a second set $G_2$ of the joints that do not contact tissue. The first set $G_1$ comprises joints in proximity to electrodes 26 determined to contact tissue from the impedance measurements; the second set $G_2$ comprises the remaining joints of probe 13A. A given electrode 26 may be determined to contact tissue according to the impedance between the electrode and a body surface electrode, such as patch 38, as is noted above. U. S. Patent 10,398,348 describes a method for determining electrode contact using impedance.

A joint may be assumed to contact tissue if it lies between two contacting electrodes, and may be assumed not to contact tissue if it lies between two non-contacting electrodes. If the joint lies between a contacting and a non-contacting electrode, it is assumed to have the property (contacting or non-contacting) of the closest electrode.

In an analysis step 110 processor 22 uses the deformed shape of probe 13A, and the known spring constant j of the probe, to calculate the external forces on each of the joints contacting tissue, i.e., on each of the joints of the first set.

As illustrated in Fig. 3, joint k has the internal probe forces given by Table I acting on the joint. In Table I $L_{k-1}$ and $L_k$ are respectively the lengths of the sections connecting at joint k, i.e., sections 80B and 80C of the probe. Also in Table I angles $a_{k-1}$, $a_k$, and $a_{k+1}$ are respectively the changes in angles at joints k-1, k and k+1 when probe 13A is deformed from its unrestrained state.

Table I

| Symbol | Description | Expression |
|---|---|---|
| $F_1$ | The force resulting from joint k-1 acting as a torsion spring. The force acts orthogonal to section 80B. | $F_1 = \dfrac{j \cdot a_{k-1}}{L_{k-1}}$ |
| $F_2$ | The force resulting from joint k+1 acting as a torsion spring. The force acts orthogonal to section 80C. | $F_2 = \dfrac{j \cdot a_{k+1}}{L_k}$ |
| $F_3$ | A reaction force, corresponding to the force $\overline{F}_3$ exerted on joint k+1 from joint k. The force acts orthogonal to section 80C. | $F_3 = \dfrac{j \cdot a_k}{L_k}$ |
| $F_4$ | A reaction force, corresponding to the force $\overline{F}_4$ exerted on joint k-1 from joint k. The force acts orthogonal to section 80B. | $F_4 = \dfrac{j \cdot a_k}{L_{k-1}}$ |

In addition to the forces listed in Table I, there are forces $T_k$ and $T_{k-1}$, respectively corresponding to compression forces in sections 80C and 80B, acting on joint k. The compression forces $T_k$ and $T_{k-1}$ are also written as $F_5$ and $F_6$.

Forces $F_1$ - $F_6$ are internal probe forces acting on joint k. In addition to the internal probe forces, the probe, in its deformed state, has an external force $F_7$ acting on joint k.

In its deformed state probe 13A is in equilibrium, so that equation (1) applies for every joint of the probe.

$$F_{total}^k = F_1^k + F_2^k + F_3^k + F_4^k + F_5^k + F_6^k + F_7^k = 0 \qquad (1)$$

where $F_{total}^k$ is the total force on joint k, and k = 1, 2, ... N.

Because each force in equation (1) is a three-dimensional force, for the N joints of probe 13A there are 3N equations corresponding to equation (1). The forces $F_1^k$, $F_2^k$, $F_3^k$, and $F_4^k$ for every joint are known in magnitude and direction, since, as is shown in Table I, all factors upon which the forces depend are known. While compression forces $F_5^k$ and $F_6^k$ are known in direction for every joint, since they are along their respective sections, their magnitude is unknown. External force $F_7^k$ for every joint is unknown in both magnitude and direction.

There are consequently 3N unknowns, for the N external forces $F_7^k$, and N-1 unknowns for the compression forces $F_5^k$ and $F_6^k$, corresponding to the number of sections of probe 13A, giving a total of 4N-1 unknowns for the 3N equations corresponding to equation (1).

In a disclosed example each external force $F_7^k$ is considered as being resolved into two components normal to each other:

$$F_7^k = F_O^k + F_P^k \qquad\qquad (2)$$

where $F_P^k$ is the component parallel to the probe direction at joint k, and $F_O^k$ is the component orthogonal to the probe direction.

A line 90 illustrates the probe direction at joint k, and it will be understood that this direction corresponds to the direction of the tangent at joint k on the probe.

In a disclosed example, joints that contact tissue, i.e., those joints in set $G_1$, are assumed to have a parallel component that is minimal i.e., zero or close to zero, because the presence of fluid between a given joint and the tissue contacting the joint reduces the friction between them. In one example the magnitude of the parallel component is assumed to be 10% or less than the magnitude of the perpendicular component.

In addition, joints that do not contact tissue, i.e., joints in set $G_2$, are assumed to have the orthogonal component minimal, i.e., zero or close to zero, since there is no tissue exerting a force on a non-contacting joint. In one example the magnitude of the orthogonal component is assumed to be 10% or less than the magnitude of the parallel component.

Using these assumptions, processor 22 finds the values of $F_O^k$, $F_P^k$, $T_k$ that, for each joint k of the probe, and subject to the constraint of equation (1) being valid, minimizes expression (3):

$$\Sigma_{i\in G_1}\left|F_P^i\right|^2 + \Sigma_{i\in G_2}\left|F_O^i\right|^2 \qquad\qquad (3)$$

In other words, processor 22 solves equation (4):

$$\left\{F_P^k, F_O^k, T_k\right\} =$$

$$\underset{\left\{F_P^k, F_O^k, T_k\right\}}{\mathrm{argmin}} \left(\Sigma_{i\in G_1}\left|F_P^i\right|^2 + \Sigma_{i\in G_2}\left|F_O^i\right|^2 \,\middle|\, \left\{F_{total}^i = 0\right\}_{i=1}^{N}\right) \qquad (4)$$

Using the solutions of equation (4), processor 22 finds the values of $F_O^k$, $F_P^k$ for each value of k, k = 1, ... N, and thus, using equation (2), the value of the external force $F_7^k$ on each joint of probe 13A.

In a final, pressure determining, step 114, processor 22 interpolates the forces of all the N joints of probe 13A. For each section, between adjacent joints, processor 22 finds a pressure on the section, as a force per unit length, using the interpolated force and the known length of each section.

Since electrodes 26 are in known locations on probe 13A, processor 22 may use the pressure found in step 114 to determine the force on each electrode 26.

A given electrode 26 may be used to ablate tissue it is contacting, and the dimensions of the ablated tissue are known to depend on the force applied by the electrode. Thus, if the procedure illustrated in Fig. 1 is an ablation procedure, processor 22 may be configured to adjust the duration and the intensity of the ablation current through the electrode according to the force from the electrode on the contacted tissue, as determined in step 114, so as to achieve desired dimensions, e.g., depth and diameter, for the ablated tissue.

Alternatively or additionally, as described above, a given electrode 26 may be used to map a location of tissue it is contacting, by processor 22 evaluating an impedance between skin patches 38 and the electrode. In a disclosed example, during a mapping procedure, processor 22 only records the determined location from the signals used to calculate the impedance if the force on the electrode, as determined in step 114, is above a preset minimum.

The description above, for probe 13A, assumes that each joint joins two sections. Those having ordinary skill in the art will be able to adapt the description, *mutatis mutandis,* to include probes having joints joining more than two sections, such as probes with multiple linear sections connected at a common point, and probes with connected loops, such as assembly 28, and all such probes are assumed to be comprised within the scope of the present disclosure.

**Examples**

Example 1. A method for performing a medical procedure, the method comprising:

providing a resilient probe (13A), having a known spring constant, for insertion into a body of a patient (23), the probe comprising a plurality of electrodes (26) and at least one position sensor (29) at respective locations along the probe; recording relative location coordinates of the electrodes and the at least one position sensor when the probe is in an unrestrained state;
while the probe is in the body and deformed by contact with tissue of the patient, recording changes in the relative location coordinates of one or more of the electrodes that are in contact with the tissue and the at least one position sensor;
computing a shape of the probe in a deformed state, in response to the recorded changes;
in response to the known spring constant of the probe and the computed shape of the probe in the deformed state, computing a respective force exerted by the tissue on each of the one or more of the electrodes; and
controlling a reception or application of electrical signals through the one or more of the electrodes from or to the tissue responsively to the computed respective force on each of the electrodes.

Example 2. The method according to example 1, wherein the known spring constant comprises a torsion spring constant.

Example 3. The method according to example 1, wherein in the unrestrained state only a force due to gravity acts on the probe.

Example 4. The method according to example 1, wherein the at least one position sensor comprises at least one coil.

Example 5. The method according to example 1, wherein the at least one position sensor comprises a linear conductor attached to a length of the probe.

Example 6. The method according to example 1, and comprising, while the probe is in the body and deformed by contact with the tissue, recording changes in the relative location coordinates of one or more of the electrodes that are not in contact with the tissue.

Example 7. The method according to example 1, wherein computing the respective force exerted by the tissue on each of the one or more of the electrodes comprises identifying a plurality of locations of the probe, proximate to the one or more electrodes, to act as spring-like joints, and calculating an external force on each of the joints, and wherein each of the locations is associated with a respective tangent to the probe thereat.

Example 8. The method according to example 7, wherein calculating the external force on each of the joints comprises calculating internal probe forces on each of the joints in response to the computed shape of the probe in the deformed state, so that the external force is equal and opposite to a resultant of the internal probe forces.

Example 9. The method according to example 7, wherein calculating the external force on each of the joints comprises identifying a set of joints in contact with the tissue, wherein a first component of the external force, for each joint of the set, parallel to the respective tangent, is less than a preset value of a second component of the external force orthogonal to the respective tangent.

Example 10. The method according to example 9, wherein the preset value is 10%.

Example 11. The method according to example 9, wherein calculating the external force on each of the joints comprises identifying a further set of joints not in contact with the tissue, wherein a third component of the external force, for each joint of the further set, orthogonal to the respective tangent, is less than a further preset value of a fourth component of the external force parallel to the respective tangent.

Example 12. The method according to example 11, wherein the preset value is 10%.

Example 13. The method according to example 11, wherein calculating the external force on each of the joints comprises evaluating the external force so that an expression comprising the first component and the third component is minimized.

Example 14. A system for performing a medical procedure, comprising:

a resilient probe (13A), having a known spring constant, for insertion into a body of a patient (23), the probe comprising a plurality of electrodes (26) and at least one position sensor (29) at respective locations along the probe; and
a processor (22), configured to:

record relative location coordinates of the electrodes and the at least one position sensor when the probe is in an unrestrained state;

while the probe is in the body and deformed by contact with tissue of the patient, record changes in the relative location coordinates of one or more of the electrodes that are in contact with the tissue and the at least one position sensor;

compute a shape of the probe in a deformed state, in response to the recorded changes;

in response to the known spring constant of the probe and the computed shape of the probe in the deformed state, compute a respective force exerted by the tissue on each of the one or more of the electrodes; and

control a reception or application of electrical signals through the one or more of the electrodes from or to the tissue responsively to the computed respective force on each of the electrodes.

Example 15. The system according to example 14, wherein the known spring constant comprises a torsion spring constant.

Example 16. The system according to example 14, wherein in the unrestrained state only a force due to gravity acts on the probe.

Example 17. The system according to example 14, wherein the at least one position sensor comprises at least one coil.

Example 18. The system according to example 14, wherein the at least one position sensor comprises a linear conductor attached to a length of the probe.

Example 19. The system according to example 14, and comprising, while the probe is in the body and deformed by contact with the tissue, the processor recording changes in the relative location coordinates of one or more of the electrodes that are not in contact with the tissue.

Example 20. The system according to example 14, wherein computing the respective force exerted by the tissue on each of the one or more of the electrodes comprises identifying a plurality of locations of the probe, proximate to the one or more electrodes, to act as spring-like joints, and calculating an external force on each of the joints, and wherein each of the locations is associated with a respective tangent to the probe thereat.

Example 21. The system according to example 20, wherein calculating the external force on each of the joints comprises calculating internal probe forces on each of the joints in response to the computed shape of the probe in the deformed state, so that the external force is equal and opposite to a resultant of the internal probe forces.

Example 22. The system according to example 20, wherein calculating the external force on each of the joints comprises identifying a set of joints in contact with the tissue, wherein a first component of the external force, for each joint of the set, parallel to the respective tangent, is less than a preset value of a second component of the external force orthogonal to the respective tangent.

Example 23. The system according to example 22, wherein the preset value is 10%.

Example 24. The system according to example 14, wherein calculating the external force on each of the joints comprises identifying a further set of joints not in contact with the tissue, wherein a third component of the external force, for each joint of the further set, orthogonal to the respective tangent, is less than a further preset value of a fourth component of the external force parallel to the respective tangent.

Example 25. The system according to example 24, wherein the preset value is 10%.

Example 26. The system according to example 24, wherein calculating the external force on each of the joints comprises evaluating the external force so that an expression comprising the first component and the third component is minimized.

The examples described above are cited by way of example, and the present disclosure is not limited by what has been particularly shown and described hereinabove. Rather the scope of the disclosure includes both combinations and sub-combinations of the various features described hereinabove, as well as variations and modifications thereof which would occur to persons skilled in the art upon reading the foregoing description and which are not disclosed in the prior art.

**Claims**

1.   A system configure for use in a medical procedure, comprising:

a resilient probe (13, 13A), having a known spring constant, for insertion into a body of a patient, the probe comprising a plurality of electrodes (26, 26A, 26B) and at least one position sensor (29) at respective locations along the probe; and

a processor (22), configured to:

record relative location coordinates of the electrodes and the at least one position sensor when the probe is in an unrestrained state;

while the probe is in the body and deformed by contact with tissue of the patient, record changes in the relative location coordinates of one or more of the electrodes that are in contact with the tissue and the at least one position sensor;

compute a shape of the probe in a deformed state, in response to the recorded changes;

in response to the known spring constant of the probe and the computed shape of the probe in the deformed state, compute a respective force exerted by the tissue on each of the one or more of the electrodes; and

control a reception or application of electrical signals through the one or more of the electrodes from or to the tissue responsively to the computed respective force on each of the electrodes.

2. The system according to claim 1, wherein the known spring constant comprises a torsion spring constant.

3. The system according to claim 1, wherein in the unrestrained state only a force due to gravity acts on the probe.

4. The system according to claim 1, wherein the at least one position sensor comprises at least one coil.

5. The system according to claim 1, wherein the at least one position sensor comprises a linear conductor attached to a length of the probe.

6. The system according to claim 1, and comprising, while the probe is in the body and deformed by contact with the tissue, the processor recording changes in the relative location coordinates of one or more of the electrodes that are not in contact with the tissue.

7. The system according to claim 1, wherein computing the respective force exerted by the tissue on each of the one or more of the electrodes comprises identifying a plurality of locations of the probe, proximate to the one or more electrodes, to act as spring-like joints, and calculating an external force on each of the joints, and wherein each of the locations is associated with a respective tangent to the probe thereat.

8. The system according to claim 7, wherein calculating the external force on each of the joints comprises calculating internal probe forces on each of the joints in response to the computed shape of the probe in the deformed state, so that the external force is equal and opposite to a resultant of the internal probe forces.

9. The system according to claim 7, wherein calculating the external force on each of the joints comprises identifying a set of joints in contact with the tissue, wherein a first component of the external force, for each joint of the set, parallel to the respective tangent, is less than a preset value of a second component of the external force orthogonal to the respective tangent.

10. The system according to claim 9, wherein the preset value is 10%.

11. The system according to claim 1, wherein calculating the external force on each of the joints comprises identifying a further set of joints not in contact with the tissue, wherein a third component of the external force, for each joint of the further set, orthogonal to the respective tangent, is less than a further preset value of a fourth component of the external force parallel to the respective tangent.

12. The system according to claim 11, wherein the preset value is 10%.

13. The system according to claim 11, wherein calculating the external force on each of the joints comprises evaluating the external force so that an expression comprising the first component and the third component is minimized.

FIG. 1

100
102

RECORD PROBE SPRING CONSTANT, LOCATIONS OF ELECTRODES AND POSITION SENSORS OF PROBE, AND SHAPE OF PROBE WHEN UNRESTRAINED.
DIVIDE PROBE INTO SECTIONS CONNECTED BY JOINTS

106

INSERT PROBE TO CONTACT TISSUE.
IDENTIFY ELECTRODES CONTACTING TISSUE AND RECORD ELECTRODE LOCATIONS.
DETERMINE PROBE SHAPE WHEN DEFORMED BY TISSUE

RECORD FIRST SET OF JOINTS CONTACTING TISSUE AND SECOND SET OF JOINTS NOT CONTACTING TISSUE

110

FOR EACH JOINT CALCULATE ALL INTERNAL KNOWN FORCES AND INTERNAL FORCE COMPONENTS.

FOR EACH JOINT DECOMPOSE EXTERNAL FORCE INTO COMPONENT PARALLEL TO PROBE AND COMPONENT ORTHOGONAL TO PROBE

ASSUMING PARALLEL COMPONENT OF EXTERNAL FORCE OF FIRST SET AND ORTHOGONAL COMPONENT OF SECOND SET ARE MINIMAL, WHILE ENSURING TOTAL OF ALL FORCES AT EACH JOINT IS ZERO, CALCULATE EXTERNAL FORCE ON EACH JOINT

114

INTERPOLATE EXTERNAL FORCES TO ESTIMATE PRESSURE ON PROBE SECTIONS.
FROM PRESSURE, CALCULATE FORCE ON EACH ELECTRODE

FIG. 2

FIG. 3

EP 4 721 686 A1

Europäisches
Patentamt
European
Patent Office
Office européen
des brevets

# EUROPEAN SEARCH REPORT

**Application Number**

EP 25 20 6586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 4 382 061 A1 (BIOSENSE WEBSTER ISRAEL LTD [IL]) 12 June 2024 (2024-06-12) * paragraphs [0007], [0026], [0032], [0041] * | 1-13 | INV.<br>A61B18/14<br>A61B5/00<br>A61B17/00 |
| Y | BACK JUNGHWAN ET AL: "Catheter contact force estimation from shape detection using a real-time Cosserat rod model", 2015 IEEE/RSJ INTERNATIONAL CONFERENCE ON INTELLIGENT ROBOTS AND SYSTEMS (IROS), IEEE, 28 September 2015 (2015-09-28), pages 2037-2042, XP032831868, DOI: 10.1109/IROS.2015.7353647 [retrieved on 2015-12-11] * the whole document * | 1-13 | |
| A | US 2009/093806 A1 (GOVARI ASSAF [IL] ET AL) 9 April 2009 (2009-04-09) * figures 1-3 * | 1-13 | |
| Y | US 2021/128010 A1 (GOVARI ASSAF [IL] ET AL) 6 May 2021 (2021-05-06) * figures 4A-4B * | 4 | TECHNICAL FIELDS SEARCHED (IPC)<br><br>A61B |
| Y | KHOSHNAM MAHTA ET AL: "Modeling and Estimation of Tip Contact Force for Steerable Ablation Catheters", IEEE TRANSACTIONS ON BIOMEDICAL ENGINEERING, IEEE, USA, vol. 62, no. 5, 1 May 2015 (2015-05-01), pages 1404-1415, XP011578786, ISSN: 0018-9294, DOI: 10.1109/TBME.2015.2389615 [retrieved on 2015-04-17] * the whole document * | 7,8 | |

The present search report has been drawn up for all claims

| Place of search | Date of completion of the search | Examiner |
|---|---|---|
| The Hague | 10 February 2026 | Husselin, Stephane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 25 20 6586

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2026

| Patent document cited in search report | | Publication date | Patent family member(s) | | Publication date |
|---|---|---|---|---|---|
| EP 4382061 | A1 | 12-06-2024 | EP | 4382061 A1 | 12-06-2024 |
| | | | IL | 308966 A | 01-07-2024 |
| | | | JP | 2024083301 A | 20-06-2024 |
| | | | US | 2024189023 A1 | 13-06-2024 |
| US 2009093806 | A1 | 09-04-2009 | AU | 2008229779 A1 | 23-04-2009 |
| | | | BR | PI0805262 A2 | 15-09-2009 |
| | | | CA | 2640817 A1 | 08-04-2009 |
| | | | CA | 2918616 A1 | 08-04-2009 |
| | | | CN | 101416874 A | 29-04-2009 |
| | | | DK | 2047797 T3 | 12-05-2014 |
| | | | DK | 2363064 T3 | 14-12-2015 |
| | | | DK | 2476371 T3 | 28-10-2013 |
| | | | EP | 2047797 A2 | 15-04-2009 |
| | | | EP | 2363064 A1 | 07-09-2011 |
| | | | EP | 2476371 A1 | 18-07-2012 |
| | | | ES | 2436361 T3 | 30-12-2013 |
| | | | ES | 2467415 T3 | 12-06-2014 |
| | | | ES | 2562706 T3 | 07-03-2016 |
| | | | IL | 194542 A | 24-03-2013 |
| | | | IL | 224115 A | 28-11-2013 |
| | | | JP | 5295707 B2 | 18-09-2013 |
| | | | JP | 5701934 B2 | 15-04-2015 |
| | | | JP | 2009090114 A | 30-04-2009 |
| | | | JP | 2013208475 A | 10-10-2013 |
| | | | KR | 20090036075 A | 13-04-2009 |
| | | | US | 2009093806 A1 | 09-04-2009 |
| | | | US | 2013096551 A1 | 18-04-2013 |
| US 2021128010 | A1 | 06-05-2021 | AU | 2017258870 A1 | 28-06-2018 |
| | | | CA | 2987756 A1 | 13-06-2018 |
| | | | CN | 108209903 A | 29-06-2018 |
| | | | EP | 3335626 A1 | 20-06-2018 |
| | | | EP | 4643807 A2 | 05-11-2025 |
| | | | IL | 283206 A | 30-06-2021 |
| | | | JP | 7080626 B2 | 06-06-2022 |
| | | | JP | 2018094417 A | 21-06-2018 |
| | | | US | 2018160936 A1 | 14-06-2018 |
| | | | US | 2021128010 A1 | 06-05-2021 |
| | | | US | 2025082220 A1 | 13-03-2025 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 11596324 B
- US 10398348 B